# EUROPEAN PATENT SPECIFICATION

(11) **EP 2 571 467 B1**
(45) Date of publication and mention of the grant of the patent: **18.10.2017**
(21) Application number: 11722119.2
(22) Date of filing: 21.04.2011
(51) Int. Cl.: A61F 13/02

(54) **WOUND PROTECTION**
WUNDSCHUTZ
PROTÈGE-PLAIE

(30) Priority: 19.05.2010 GB 201008347
(43) Date of publication of application: 27.03.2013
(73) Proprietor: Smith & Nephew PLC, London WC2N 6LA (GB)
(72) Inventor: ADIE, Gordon Campbell, York YO10 5DF (GB); COLLINSON, Sarah Jenny, York YO10 5DF (GB); FRYER, Christopher John, York YO10 5DF (GB); PERON, Yannick Louis, York YO10 5DF (GB); NICOLINI, Derek, York YO10 5DF (GB); HARTWELL, Edward Yerbury, York YO10 5DF (GB)
(74) Representative: Smith & Nephew
(86) International application number: PCT/GB2011/000621
(87) International publication number: WO 2011/144888

(56) References cited:
- EP-A1- 0 617 152
- WO-A1-95/16416
- WO-A1-2006/087021
- WO-A1-2008/104609
- WO-A1-2009/066105
- US-A1- 2009 234 307

## Description

The present invention relates to a method and apparatus for providing protection to a wound site. In particular, but not exclusively, the present invention relates to a wound dressing useable during topical negative pressure (TNP) therapy in which the wound dressing acts as a buffer to help prevent compression or shear forces exerted on the wound dressing, for example due to patient movement, from harming a healing wound.

There is much prior art available relating to the provision of apparatus and methods of use thereof for the application of topical negative pressure (TNP) therapy to wounds together with other therapeutic processes intended to enhance the effects of the TNP therapy. Examples of such prior art include those listed and briefly described below.

WO 2006/087021 Fleischmann discloses a wound treatment device comprising a wound surface contacting plug.

TNP therapy assists in the closure and healing of wounds by reducing tissue oedema; encouraging blood flow; stimulating the formation of granulation tissue; removing excess exudates and may reduce bacterial load and thus, infection to the wound. Furthermore, TNP therapy permits less outside disturbance of the wound and promotes more rapid healing.

Certain prior art apparatus and methods are generally only applicable to a patient when hospitalised as the apparatus used is complex, needing people having specialist knowledge in how to operate and maintain the apparatus, and also relatively heavy and bulky, not being adapted for easy mobility outside of a hospital environment by a patient, for example.

Some patients having relatively less severe wounds which do not require continuous hospitalisation, for example, but whom nevertheless would benefit from the prolonged application of TNP therapy, could be treated at home or at work subject to the availability of an easily portable and maintainable TNP therapy apparatus. To this end it is known to provide a portable TNP therapy unit which may be carried by a patient and clipped to belt or harness. A negative pressure can thus be applied at a wound site.

During TNP therapy a portable or non-portable therapy unit generates a negative pressure at a wound site. As fluid, including air as well as wound exudate material is removed from the wound site this must be collected in some manner remote from the wound site. With prior known therapy units the collection and storage of wound exudate material is typically carried out by a waste canister connected to a pump unit of the
therapy unit. The use of a canister, however, can result in the therapy unit apparatus itself being quite bulky and expensive to manufacture. Also replacing a canister or a bag in a canister in which wound exudate is collected can be a time consuming and relatively unhygienic process.

It will be appreciated that from time to time accidents may happen to patients undergoing negative pressure wound therapy. Such accidents might cause short term or long term forces to be applied to a dressing covering a wound. Alternatively patient movement may bring the patient and any dressing covering a healing wound into contact with an external object. In such occurrences compressive forces or lateral forces may occur. Such force can cause disturbance of a wound bed which can damage a wound site. A particular cause for concern is during the treatment of skin graft wounds. Under such conditions lateral forces can entirely upset or tear apart a healing skin graft region.

It is an aim of the present invention to at least partly mitigate the above-mentioned problems.

It is an aim of certain embodiments of the present invention to provide a method for providing negative pressure at a wound site to aid in wound closure and healing in which wound exudate drawn from a wound site during the therapy is collected and stored in a wound dressing.

It is an aim of certain embodiments of the present invention to provide a wound dressing having an increased capacity to absorb compressive forces exerted on the wound dressing.

It is an aim of certain embodiments of the present invention to provide a wound dressing having an increased capacity to prevent shear forces from an outer surface of a wound dressing from being translated into corresponding shear forces at a wound site.

It is an aim of certain embodiments of the present invention to provide a wound dressing which can "give" in a direction perpendicular to and parallel to a wound site surface even when the dressing experiences negative pressure.

The invention is as claimed in the claims.

Certain embodiments provide a wound dressing which even when under negative pressure conditions is able to provide further "give" to buffer compression forces from harming a wound.

Certain embodiments of the present invention provide a wound dressing able to disconnect shear forces applied to the dressing from the wound site covered by the dressing. As a result damage to the wound can be wholly or at least partially avoided.

Certain embodiments provide the advantage that a wound site can be covered with a wound dressing which is simultaneously able to deliver negative pressure wound therapy to a wound site, collect exudate and provide protection from forces operating on the dressing.

Certain embodiments of the present invention provide the advantage that forces operating on a dressing can be offset by dissipating loads operating over a relatively small distance on an upper layer of the dressing to a relatively larger area on a lower surface of the dressing. The force is thus dissipated over a larger area thus reducing the effect of the force.

Embodiments of the present invention will now be described hereinafter, by way of example only, with reference to the accompanying drawings in which:
Figure 1 illustrates a wound dressing;
Figure 2 illustrates a top view of a wound dressing;
Figure 3 illustrates a top view of a wound dressing including baffle elements;
Figure 4 illustrates a top view of a further wound dressing including baffle elements;
Figure 5 illustrates a baffle element according to one embodiment;
Figure 6 illustrates a top view of a wound dressing including a single baffle element;
Figure 7 illustrates a top view of a wound dressing including an air channel;
Figure 8 illustrates a top view of a wound dressing including two air channels;
Figure 9 illustrates a top view of a wound dressing including two orifices in a cover layer coupled through a fluid communication passage;
Figure 10 illustrates an embodiment of the fluid communication passage;
Figure 11 illustrates a top view of a suction port;
Figure 12 illustrates a suction port including a filter element;
Figure 13 illustrates a further suction port including a filter element; and
Figure 14 illustrates a range of exemplary configurations of baffle elements in a wound dressing;
Figure 15 illustrates an exemplary configuration of vias in a transmission layer of a wound dressing;
Figure 16 illustrates a top view of a wound dressing including an elongate orifice in a cover layer;
Figure 17 illustrates a transmission layer in a relaxed mode of operation;
Figure 18 illustrates a transmission layer in a forced mode of operation;
Figure 19 illustrates pressure offsetting;
Figure 20 illustrates a transmission layer and overlying absorbent layer in a relaxed mode of operation;
Figure 21 illustrates an absorbent layer and transmission layer experiencing a compressive force;
Figure 22 illustrates an absorbent layer and transmission layer experiencing a shear force;
Figure 23 illustrates an embodiment of a wound treatment system; and,
Figures 24A-D illustrate the use and application of an embodiment of a wound treatment system onto a patient.

In the drawings like reference numerals refer to like parts.

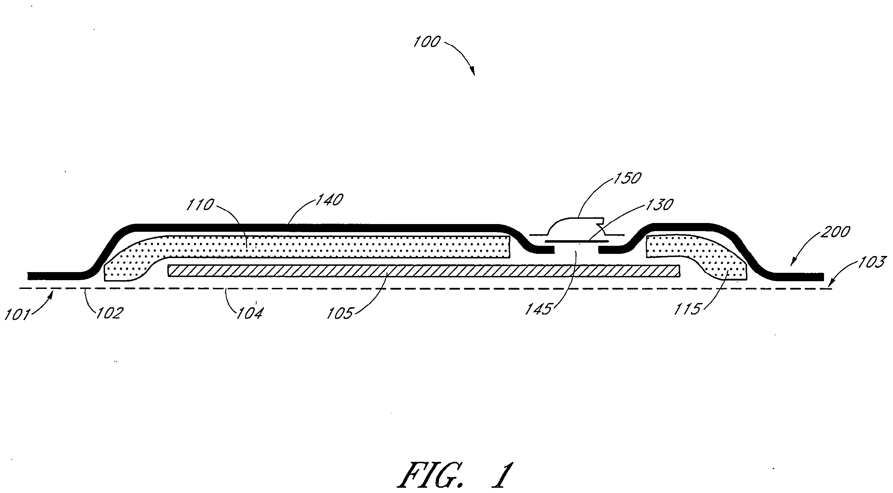

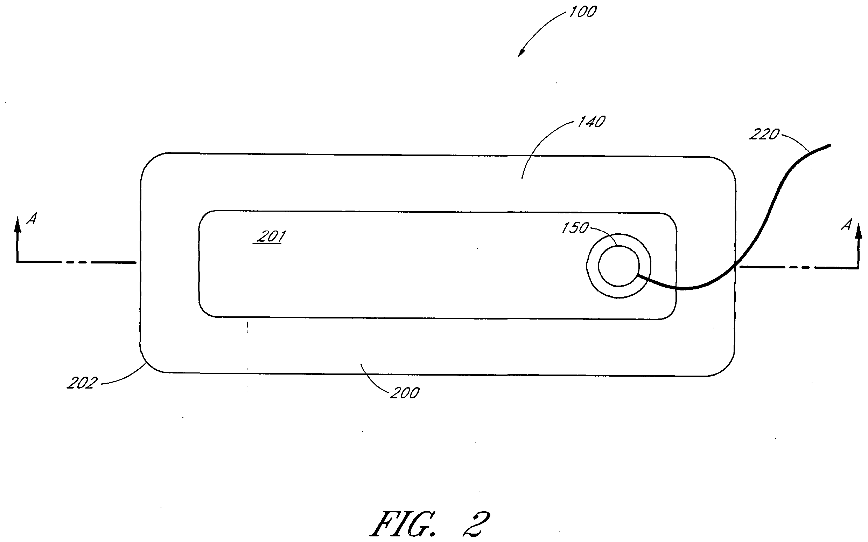

Figure 1 illustrates a cross section through a wound dressing 100 according to an embodiment of the invention. A plan view from above the wound dressing 100 is illustrated in Figure 2 with the line A-A indicating the location of the cross section shown in Figure 1. It will be understood that Figure 1 illustrates a generalised schematic view of an apparatus 100. It will be understood that embodiments of the present invention are generally applicable to use in topical negative pressure (TNP) systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP systems may also assist on the healing of surgically closed wounds by removing fluid and by helping to stabilise the tissue in the apposed position of closure. A further beneficial use of TNP can be found in grafts and flaps where removal of excess fluid is important and close proximity of the graft to tissue is required in order to ensure tissue viability.

The wound dressing 100 can be located over a wound site to be treated. The dressing 100 forms a sealed cavity over the wound site. It will be appreciated that throughout this specification reference is made to a wound. In this sense it is to be understood that the term wound is to be broadly construed and encompasses open and closed wounds in which skin is torn, cut or punctured or where trauma causes a contusion. A wound is thus broadly defined as any damaged region of tissue where fluid may or may not be produced. Examples of such wounds include, but are not limited to, incisions, lacerations, abrasions, contusions, burns, diabetic ulcers, pressure ulcers, stoma, surgical wounds, trauma and venous ulcers or the like.

In some embodiments, it may be preferable for the wound site to be filled partially or completely with a wound packing material. This wound packing material is optional, but may be desirable in certain wounds, for example deeper wounds. The wound packing material can be used in addition to the wound dressing 100. The wound packing material generally may comprise a porous and conformable material, for example foam (including reticulated foams), and gauze. Preferably, the wound packing material is sized or shaped to fit within the wound site so as to fill any empty spaces. The wound dressing 100 may then be placed over the wound site and wound packing material overlying the wound site. When a wound packing material is used, once the wound dressing 100 is sealed over the wound site, TNP is transmitted from a pump through the wound dressing 100, through the wound packing material, and to the wound site. This negative pressure draws wound exudate and other fluids or secretions away from the wound site.

It is envisaged that the negative pressure range for the apparatus embodying the present invention may be between about -20 mmHg and -200 mmHg (note that these pressures are relative to normal ambient atmospheric pressure thus, -200 mmHg would be about 560 mmHg in practical terms). Aptly the pressure range may be between about -40 mmHg and -150 mmHg. Alternatively a pressure range of up to -75 mmHg, up to -80 mmHg or over -80 mmHg can be used. Also aptly a pressure range of below -75 mmHg could be used. Alternatively a pressure range of over -100 mmHg could be used or over -150 mmHg.

It will be appreciated that according to certain embodiments of the present invention the pressure provided may be modulated over a period of time according to one or more desired and predefined pressure profiles. For example such a profile may include modulating the negative pressure between two predetermined negative pressures P1 and P2 such that pressure is held substantially constant at P1 for a pre-determined time period T1 and then adjusted by suitable means such as varying pump work or restricting fluid flow or the like, to a new predetermined pressure P2 where the pressure may be held substantially constant for a further predetermined time period T2. Two, three or four or more predetermined pressure values and respective time periods may be optionally utilised. Aptly more complex amplitude/frequency wave forms of pressure flow profiles may also be provided eg sinusoidal, sore tooth, systolic-diastolic or the like etc.

As illustrated in Figure 1 a lower surface 101 of the wound dressing 100 is provided by an optional wound contact layer 102. The wound contact layer 102 can be a polyurethane layer or polyethylene layer or other flexible layer which is perforated, for example via a hot pin process, laser ablation process, ultrasound process or in some other way or otherwise made permeable to liquid and gas. The wound contact layer has a lower surface 101 and an upper surface 103. The perforations 104 are through holes in the wound contact layer which enables fluid to flow through the layer. The wound contact layer helps prevent tissue ingrowth into the other material of the wound dressing. The perforations are small enough to meet this requirement but still allow fluid through. For example, perforations formed as slits or holes having a size ranging from 0.025 mm to 1.2 mm are considered small enough to help prevent tissue ingrowth into the wound dressing while allowing wound exudate to flow into the dressing. The wound contact layer helps hold the whole wound dressing together and helps to create an air tight seal around the absorbent pad in order to maintain negative pressure at the wound. The wound contact layer also acts as a carrier for an optional lower and upper adhesive layer (not shown). For example, a lower pressure sensitive adhesive may be provided on the underside surface 101 of the wound dressing whilst an upper pressure sensitive adhesive layer may be provided on the upper surface 103 of the wound contact layer. The pressure, sensitive, adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilised this helps adhere the wound dressing to the skin around a wound site.

A layer 105 of porous material is located above the wound contact layer. This porous layer, or transmission layer, 105 allows transmission of fluid including liquid and gas away from a wound site into upper layers of the wound dressing. In particular, the transmission layer 105 ensures that an open air channel can be maintained to communicate negative pressure over the wound area even when the absorbent layer has absorbed substantial amounts of exudates. The layer should remain open under the typical pressures that will be applied during negative pressure wound therapy as described above, so that the whole wound site sees an equalised negative pressure. The layer 105 is formed of a material having a three dimensional structure that could comprise an open celled foam, a knitted or woven spacer fabric (for example Baltex 7970 weft knitted polyester) or a non-woven fabric.

Aptly, the transmission layer comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 100 denier flat polyester and a third layer formed sandwiched between these two layers which is a region defined by a knitted polyester viscose, cellulose or the like monofilament fibre. Other materials and other linear mass densities of fibre could of course be used.

Whilst reference is made throughout this disclosure to a monofilament fibre it will be appreciated that a multistrand alternative could of course be utilised.

The top spacer fabric thus has more filaments in a yarn used to form it than the number of filaments making up the yarn used to form the bottom spacer fabric layer. This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent layer helps lock the liquid away or itself wicks the liquid onwards towards the cover layer where it can be transpired.

Aptly, to improve the liquid flow across the transmission layer (that is to say perpendicular to the channel region formed between the top and bottom spacer layers, the 3D fabric is treated with a dry cleaning agent (such as, but not limited to, Per Chloro Ethylene) to help remove any manufacturing products such as mineral oils, fats and/or waxes used previously which might interfere with the hydrophilic capabilities of the transmission layer. Aptly, an additional manufacturing step can subsequently be carried in which the 3D spacer fabric is washed in a hydrophilic agent (such as, but not limited to, Feran Ice 30g/l available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

A layer 110 of absorbent material is provided above the transmission layer 105. The absorbent material which may be a foam or non-woven natural or synthetic material and which may optionally include or be super-absorbent material forms a reservoir for fluid, particularly liquid, removed from the wound site and draws those fluids towards a cover layer 140. The material of the absorbent layer also prevents liquid collected in the wound dressing from flowing in a sloshing manner. The absorbent layer 110 also helps distribute fluid throughout the layer via a wicking action so that fluid is drawn from the wound site and stored throughout the absorbent layer. This prevents agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 110 may typically be manufactured from ALLEVYN^{™} foam, Freudenberg 114-224-4 and/or Chem-Posite™11C-450.

Aptly, the absorbent layer is a layer of non-woven cellulose fibres having super-absorbent material in the form of dry particles dispersed throughout. Use of the cellulose fibres introduces fast wicking elements which help quickly and evenly distribute liquid taken up by the dressing. The juxtaposition of multiple strand-like fibres leads to strong capillary action in the fibrous pad which helps distribute liquid. In this way, the super-absorbent material is efficiently supplied with liquid. Also, all regions of the absorbent layer are provided with liquid.

The wicking action also assists in bringing liquid into contact with the upper cover layer to aid increase transpiration rates of the dressing.

The wicking action also assists in delivering liquid downwards towards the wound bed when exudation slows or halts. This delivery process helps maintain the transmission layer and lower wound bed region in a moist state which helps prevent crusting within the dressing (which could lead to blockage) and helps maintain an environment optimised for wound healing.

Aptly, the absorbent layer may be an air-laid material. Heat fusible fibres may optionally be used to assist in holding the structure of the pad together. It will be appreciated that rather than using super-absorbing particles or in addition to such use, super-absorbing fibres may be utilised according to certain embodiments of the present invention. An example of a suitable material is the Product Chem-Posite^{™} 11 C available from Emerging Technologies Inc (ETi) in the USA.

Optionally, according to certain embodiments of the present invention, absorbent layer may include synthetic stable fibres and/or bi-component stable fibres and/or natural stable fibres and/or super-absorbent fibres. Fibres in the absorbent layer may be secured together by latex bonding or thermal bonding or hydrogen bonding or a combination of any bonding technique or other securing mechanism. Aptly, the absorbent layer is formed by fibres which operate to lock super-absorbent particles within the absorbent layer. This helps ensure that super-absorbent particles do not move external to the absorbent layer and towards an underlying wound bed. This is particularly helpful because when negative pressure is applied there is a tendency for the absorbent pad to collapse downwards and this action would push super-absorbent particle matter into a direction towards the wound bed if they were not locked away by the fibrous structure of the absorbent layer.

The absorbent layer comprises a layer of multiple fibres. Aptly, the fibres are strand-like and made from cellulose, polyester, viscose or the like. Aptly, dry absorbent particles are distributed throughout the absorbent layer ready for use. Aptly, the absorbent layer comprises a pad of cellulose fibres and a plurality of super absorbent particles. Aptly, the absorbent layer is a non-woven layer of randomly orientated cellulose fibres.

Super-absorber particles/fibres may be, for example, sodium polyacrylate or carbomethoxycellulose materials or the like or any material capable of absorbing many times its own weight in liquid. Aptly, the material can absorb more than five times its own weight of 0.9% WNV saline, etc. Aptly, the material can absorb more than 15 times its own weight of 0.9% W/W saline, etc. Aptly, the material is capable of absorbing more than 20 times its own weight of 0.9% W/W saline, etc. Aptly, the material is capable of absorbing more than 30 times its own weight of 0.9% W/W saline, etc.

Aptly, the particles of superabsorber are very hydrophilic and grab the fluid as it enters the dressing, swelling up on contact. An equilibrium is set up within the dressing core whereby moisture passes from the superabsorber into the dryer surrounding area and as it hits the top film the film switches and the fluid vapour starts to be transpired. A moisture gradient is established within the dressing to continually remove fluid from the wound bed and ensure the dressing does not become heavy with exudate.

Aptly the absorbent layer includes at least one through hole located so as to underly the suction port. As illustrated in Figure 1 a single through hole can be used to produce an opening underlying the port 150. It will be appreciated that multiple openings could alternatively be utilised. Additionally should more than one port be utilised according to certain embodiments of the present invention one or multiple openings may be made in the super-absorbent layer in registration with each respective port. Although not essential to certain embodiments of the present invention the use of through holes in the super-absorbent layer provide a fluid flow pathway which is particularly unhindered and this is useful in certain circumstances.

Where an opening is provided in the absorbent layer the thickness of the layer itself will act as a stand-off separating any overlying layer from the upper surface (that is to say the surface facing away from a wound in use) of the transmission layer 105. An advantage of this is that the filter of the port is thus decoupled from the material of the transmission layer. This helps reduce the likelihood that the filter will be wetted out and thus will occlude and block further operation.

Use of one or more through holes in the absorption layer also has the advantage that during use if the absorbent layer contains a gel forming material, such as superabsorber, that material as it expands to absorb liquid, does not form a barrier through which further liquid movement and fluid movement in general cannot pass. In this way each opening in the absorbent layer provides a fluid pathway between the transmission layer directly to the wound facing surface of the filter and then onwards into the interior of the port.

A gas impermeable, but moisture vapour permeable, cover layer 140 extends across the width of the wound dressing. The cover layer, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way an effective chamber is made between the cover layer and a wound site where a negative pressure can be established. The cover layer 140 is sealed to the wound contact layer 102 in a border region 200 around the circumference of the dressing, ensuring that no air is drawn in through the border area, for example via adhesive or welding techniques. The cover layer 140 protects the wound from external bacterial contamination (bacterial barrier) and allows liquid from wound exudates to be transferred through the layer and evaporated from the film outer surface. The cover layer 140 typically comprises two layers; a polyurethane film and an adhesive pattern spread onto the film. The polyurethane film is moisture vapour permeable and may be manufactured from a material that has an increased water transmission rate when wet.

The absorbent layer 110 may be of a greater area than the transmission layer 105, as illustrated in Figure 1, such that the absorbent layer overlaps the edges of the transmission layer 105, thereby ensuring that the transmission layer does not contact the cover layer 140. This provides an outer channel 115 of the absorbent layer 110 that is in direct contact with the wound contact layer 102, which aids more rapid absorption of exudates to the absorbent layer. Furthermore, this outer channel 115 ensures that no liquid is able to pool around the circumference of the wound cavity, which may otherwise seep through the seal around the perimeter of the dressing leading to the formation of leaks.

In order to ensure that the air channel remains open when a vacuum is applied to the wound cavity, the transmission layer 105 must be sufficiently strong and non-compliant to resist the force due to the pressure differential. However, if this layer comes into contact with the relatively delicate cover layer 140, it can cause the formation of pin-hole openings in the cover layer 140 which allow air to leak into the wound cavity. This may be a particular problem when a switchable type polyurethane film is used that becomes weaker when wet. The absorbent layer 110 is generally formed of a relatively soft, non-abrasive material compared to the material of the transmission layer 105 and therefore does not cause the formation of pin-hole openings in the cover layer. Thus by providing an absorbent layer 110 that is of greater area than the transmission layer 105 and that overlaps the edges of the transmission layer_105, contact between the transmission layer and the cover layer is prevented, avoiding the formation of pin-hole openings in the cover layer 140.

The absorbent layer 110 is positioned in contact with the cover layer 140. As the absorbent layer absorbs wound exudate, the exudate is drawn towards the cover layer 140, bringing the water component of the exudate into contact with the moisture vapour permeable cover layer. This water component is drawn into the cover layer itself and then evaporates from the top surface of the dressing. In this way, the water content of the wound exudate can be transpired from the dressing, reducing the volume of the remaining wound exudate that is to be absorbed by the absorbent layer 110, and increasing the time before the dressing becomes full and must be changed. This process of transpiration occurs even when negative pressure has been applied to the wound cavity, and it has been found that the pressure difference across the cover layer when a negative pressure is applied to the wound cavity has negligible impact on the moisture vapour transmission rate across the cover layer.

An orifice 145 is provided in the cover film 140 to allow a negative pressure to be applied to the dressing 100. A suction port 150 is sealed to the top of the cover film 140 over the orifice 145, and communicates negative pressure through the orifice 145. A length of tubing 220 may be coupled at a first end to the suction port 150 and at a second end to a pump unit (not shown) to allow fluids to be pumped out of the dressing. The port may be adhered and sealed to the cover film 140 using an adhesive such as an acrylic, cyanoacrylate, epoxy, UV curable or hot melt adhesive. The port 150 is formed from a soft polymer, for example a polyethylene, a polyvinyl chloride, a silicone or polyurethane having a hardness of 30 to 90 on the Shore A scale.

An aperture is provided in the absorbent layer 110 beneath the orifice 145 such that the orifice is connected directly to the transmission layer 105. This allows the negative pressure applied to the port 150 to be communicated to the transmission layer 105 without passing through the absorbent layer 110. This ensures that the negative pressure applied to the wound site is not inhibited by the absorbent layer as it absorbs wound exudates. In other embodiments, no aperture may be provided in the absorbent layer 110, or alternatively a plurality of apertures underlying the orifice 145 may be provided.

As shown in Figure 1, one embodiment of the wound dressing 100 comprises an aperture in the absorbent layer 100 situated underneath the port 150. In use, for example when negative pressure is applied to the dressing 100, a wound facing portion of the port 150 may thus come into contact with the transmission layer 105, which can thus aid in transmitting negative pressure to the wound site even when the absorbent layer 110 is filled with wound fluids. Some embodiments may have the cover layer 140 be at least partly adhered to the transmission layer 105. In some embodiments, the aperture is at least 1-2 mm larger than the diameter of the port 150, or the orifice 145.

A filter element 130 that is impermeable to liquids, but permeable to gasses is provided to act as a liquid barrier, and to ensure that no liquids are able to escape from the wound dressing. The filter element may also function as a bacterial barrier. Typically the pore size is 0.2µm. Suitable materials for the filter material of the filter element 130 include 0.2 micron Gore™ expanded PTFE from the MMT range, PALL Versapore™ 200R, and Donaldson™ TX6628. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter. The filter element can be attached or sealed to the port and/or the cover film 140 over the orifice 145. For example, the filter element 130 may be moulded into the port 150, or may be adhered to both the top of the cover layer 140 and bottom of the port 150 using an adhesive such as a UV cured adhesive.

It will be understood that other types of material could be used for the filter element 130. More generally a microporous membrane can be used which is a thin, flat sheet of polymeric material, this contains billions of microscopic pores. Depending upon the membrane chosen these pores can range in size from 0.01 to more than 10 micrometers. Microporous membranes are available in both hydrophilic (water filtering) and hydrophobic (water repellent) forms. In some embodiments of the invention, filter element 130 comprises a support layer and an acrylic co-polymer membrane formed on the support layer. Aptly the wound dressing 100 according to certain embodiments of the present invention uses microporous hydrophobic membranes (MHMs). Numerous polymers may be employed to form MHMs. For example, PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing air to flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter element 130 may also include an odour absorbent material, for example activated charcoal, carbon fibre cloth or Vitec Carbotec-RT Q2003073 foam, or the like. For example, an odour absorbent material may form a layer of the filter element 130 or may be sandwiched between microporous hydrophobic membranes within the filter element.

The filter element 130 thus enables gas to be exhausted through the orifice 145. Liquid, particulates and pathogens however are contained in the dressing.

In particular for embodiments with a single port 150 and through hole, it may be preferable for the port 150 and through hole to be located in an off-center position as illustrated in Figures 1 and 2. Such a location may permit the dressing 100 to be positioned onto a patient such that the port 150 is raised in relation to the remainder of the dressing 100. So positioned, the port 150 and the filter 130 may be less likely to come into contact with wound fluids that could prematurely occlude the filter 130 so as to impair the transmission of negative pressure to the wound site.

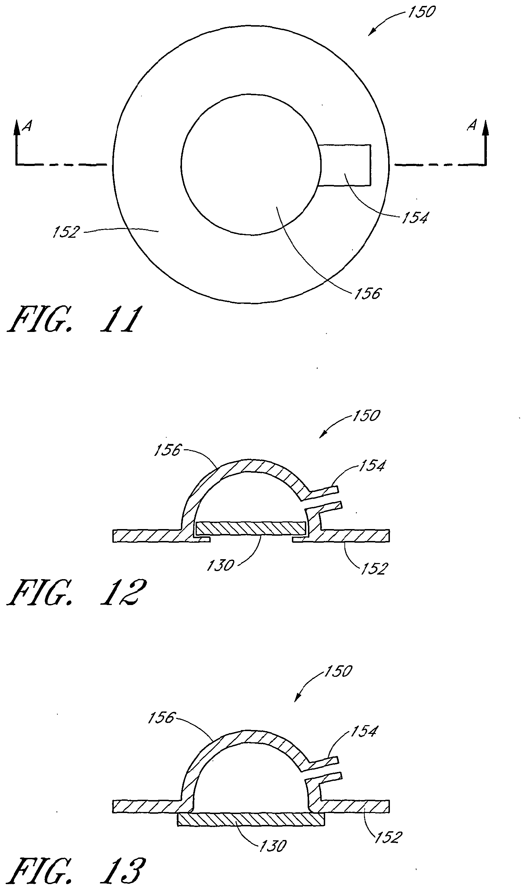

Figure 11 shows a plan view of a suction port 150 according to some embodiments of the invention. The suction port comprises a sealing surface 152 for sealing the port to a wound dressing, a connector portion 154 for connecting the suction port 150 to a source of negative pressure, and a hemispherical body portion 156 disposed between the sealing surface 152 and the connector portion 154. Sealing surface 152 comprises a flange that provides a substantially flat area to provide a good seal when the port 150 is sealed to the cover layer 140. Connector portion 154 is arranged to be coupled to the external source of negative pressure via a length of tube 220.

According to some embodiments, the filter element 130 forms part of the bacterial barrier over the wound site, and therefore it is important that a good seal is formed and maintained around the filter element. However, it has been determined that a seal formed by adhering the filter element 130 to the cover layer 140 is not sufficiently reliable. This is a particular problem when a moisture vapour permeable cover layer is used, as the water vapour transpiring from the cover layer 140 can affect the adhesive, leading to breach of the seal between the filter element and the cover layer. Thus, according to some embodiments of the invention an alternative arrangement for sealing the filter element 130 to stop liquid from entering the connector portion 154 is employed.

Figure 12 illustrates a cross section through the suction port 150 of Figure 11 according to some embodiments of the invention, the line A-A in Figure 11 indicating the location of the cross section. In the suction port of Figure 12, the suction port 150 further comprises filter element 130 arranged within the body portion 156 of the suction port 150. A seal between the suction port 150 and the filter element 130 is achieved by moulding the filter element within the body portion of the suction port 150.

Figure 13 illustrates a cross section through the suction port 150 of Figure 11 according to some embodiments of the invention. In the suction port of Figure 13, the filter element 130 is sealed to the sealing surface 152 of the suction port 150. The filter element may be sealed to the sealing surface using an adhesive or by welding the filter element to the sealing surface.

By providing the filter element 130 as part of the suction port 150, as illustrated in Figures 12 and 13, the problems associated with adhering the filter element to the cover layer 140 are avoided allowing a reliable seal to be provided. Furthermore, providing a sub-assembly having the filter element 130 included as part of the suction port 150 allows for simpler and more efficient manufacture of the wound dressing 100.

While the suction port 150 has been described in the context of the wound dressing 100 of Figure 1, it will be understood that the embodiments of Figures 12 and 13 are applicable to any wound dressing for applying a negative pressure to a wound, wherein wound exudate drawn from the wound is retained within the dressing. According to some embodiments of the invention, the suction port 150 may be manufactured from a transparent material in order to allow a visual check to be made by a user for the ingress of wound exudate into the suction port 150.

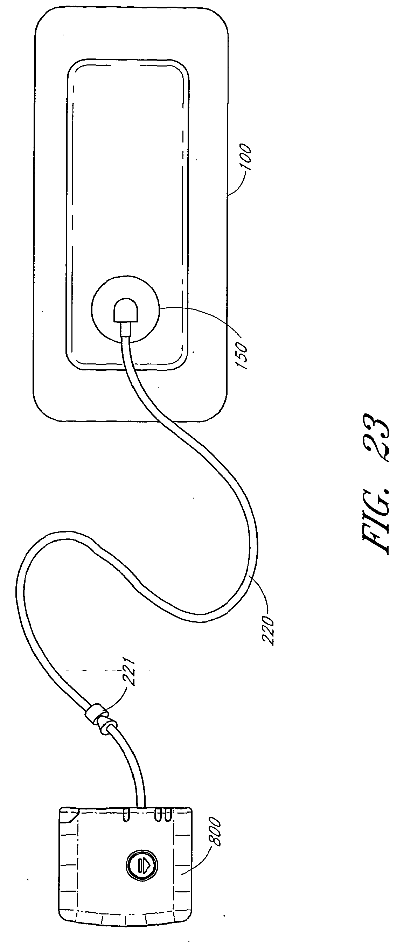

In operation the wound dressing 100 is sealed over a wound site forming a wound cavity. A pump unit (illustrated in Figure 23 and described in further detail below) applies a negative pressure at a connection portion 154 of the port 150 which is communicated through the orifice 145 to the transmission layer 105. Fluid is drawn towards the orifice through the wound dressing from a wound site below the wound contact layer 102. The fluid moves towards the orifice through the transmission layer 105. As the fluid is drawn through the transmission layer 105 wound exudate is absorbed into the absorbent layer 110.

Turning to Figure 2 which illustrates a wound dressing 100 in accordance with an embodiment of the present invention one can see the upper surface of the cover layer 140 which extends outwardly away from a centre of the dressing into a border region 200 surrounding a central raised region 201 overlying the transmission layer 105 and the absorbent layer 110. As indicated in Figure 2 the general shape of the wound dressing is rectangular with rounded corner regions 202. It will be appreciated that wound dressings according to other embodiments of the present invention can be shaped differently such as square, circular or elliptical dressings, or the like.

The wound dressing 100 may be sized as necessary for the size and type of wound it will be used in. In some embodiments, the wound dressing 100 may measure between 20 and 40 cm on its long axis, and between 10 to 25 cm on its short axis. For example, dressings may be provided in sizes of 10 x 20 cm, 10 x 30 cm, 10 x 40 cm, 15 x 20 cm, and 15 x 30 cm. In some embodiments, the wound dressing 100 may be a square-shaped dressing with sides measuring between 15 and 25 cm (e.g., 15 x 15 cm, 20 x 20 cm and 25 x 25 cm. The absorbent layer 110 may have a smaller area than the overall dressing, and in some embodiments may have a length and width that are both about 3 to 10 cm shorter, more preferably about 5 cm shorter, than that of the overall dressing 100. In some rectangular-shape embodiments, the absorbent layer 110 may measure between 15 and 35 cm on its long axis, and between 5 and 10 cm on its short axis. For example, absorbent layers may be provided in sizes of 5.6 x 15 cm (for 10 x 20 cm dressings), 5.6 x 25 cm (for 10 x 30 cm dressings), 5.6 x 35 cm (for 10 x 40 cm dressings), 10 x 15 cm (for 15 x 20 cm dressings), and 10 x 25 cm (for 15 x 30 cm dressings). In some square-shape embodiments, the absorbent layer 110 may have sides that are between 10 and 20 cm in length (e.g., 10 x 10 cm for a 15 x 15 cm dressing, 15 x 15 cm for a 20 x 20 cm dressing, or 20 x 20 cm for a 25 x 25 cm dressing). The transmission layer 105 is preferably smaller than the absorbent layer, and in some embodiments may have a length and width that are both about 0.5 to 2 cm shorter, more preferably about 1 cm shorter, than that of the absorbent layer. In some rectangular-shape embodiments, the transmission layer may measure between 14 and 34 cm on its long axis and between 3 and 5 cm on its short axis. For example, transmission layers may be provided in sizes of 4.6 x 14 cm (for 10 x 20 cm dressings), 4.6 x 24 cm (for 10 x 30 cm dressings), 4 x 34 cm (for 10 x 40 cm dressings), 9 x 14 cm (for 15 x 20 cm dressings), and 9 x 24 cm (for 15 x 30 cm dressings). In some square-shape embodiments, the transmission layer may have sides that are between 9 and 19 cm in length (e.g.,- 9 x 9 cm for a 15 x 15 cm dressing, 14 x 14 cm for a 20 x 20 cm dressing, or 19 x 19 cm for a 25 x 25 cm dressing).

It will be understood that according to embodiments of the present invention the wound contact layer is optional. This layer is, if used, porous to water and faces an underlying wound site. A transmission layer 105 such as an open celled foam, or a knitted or woven spacer fabric is used to distribute gas and fluid removal such that all areas of a wound are subjected to equal pressure. The cover layer together with the filter layer forms a substantially liquid tight seal over the wound. Thus when a negative pressure is applied to the port 150 the negative pressure is communicated to the wound cavity below the cover layer. This negative pressure is thus experienced at the target wound site. Fluid including air and wound exudate is drawn through the wound contact layer and transmission layer 105. The wound exudate drawn through the lower layers of the wound dressing is dissipated and absorbed into the absorbent layer 110 where it is collected and stored. Air and moisture vapour is drawn upwards through the wound dressing through the filter layer and out of the dressing through the suction port. A portion of the water content of the wound exudate is drawn through the absorbent layer and into the cover layer 140 and then evaporates from the surface of the dressing.

As discussed above, when a negative pressure is applied to a wound dressing sealed over a wound site, fluids including wound exudate are drawn from the wound site and through the transmission layer 105 towards the orifice 145. Wound exudate is then drawn into the absorbent layer 110 where it is absorbed. However, some wound exudate may not be absorbed and may move to the orifice 145. Filter element 130 provides a barrier that stops any liquid in the wound exudate from entering the connection portion 154 of the suction port 150. Therefore, unabsorbed wound exudate may collect underneath the filter element 130. If sufficient wound exudate collects at the filter element, a layer of liquid will form across the surface of filter element 130 and the filter element will become blocked as the liquid cannot pass through the filter element 130 and gases will be stopped from reaching the filter element by the liquid layer. Once the filter element becomes blocked, negative pressure can no longer be communicated to the wound site, and the wound dressing must be changed for a fresh dressing, even though the total capacity of the absorbent layer has not been reached.

In a preferred embodiment, the port 150, along with any aperture 146 in the absorbing layer 110 situated below it, generally aligns with the mid-longitudinal axis A-A illustrated in Figure 2. Preferably, the port 150 and any such aperture 146 are situated closer to one end of the dressing, contrasted with a central position. In some embodiments, the port may be located at a corner of the dressing 100. For example, in some rectangular embodiments, the port 150 may be located between 4 and 6 cm from the edge of the dressing, with the aperture 146 located 2 to 3 cm from the edge of the absorbent layer. In some square embodiments, the port 150 may be located between 5 to 8 cm from the corner of the dressing, with the aperture 146 located 3 to 5 cm from the corner of the absorbent layer.

Certain orientations of the wound dressing may increase the likelihood of the filter element 130 becoming blocked in this way, as the movement of the wound exudate through the transmission layer may be aided by the effect of gravity. Thus, if due to the orientation of the wound site and wound dressing, gravity acts to increase the rate at which wound exudate is drawn towards the orifice 145, the filter may become blocked with wound exudate more quickly. Thus, the wound dressing would have to be changed more frequently and before the absorbent capacity of the absorbent layer 110 has been reached.

In order to avoid the premature blocking of the wound dressing 100 by wound exudate drawn towards the orifice 145 some embodiments of the invention include at least one element configured to reduce the rate at which wound exudate moves towards the orifice 145. The at least one element may increase the amount of exudate that is absorbed into the absorbent layer before reaching the orifice 145 and/or may force the wound exudate to follow a longer path through the dressing before reaching the orifice 145, thereby increasing the time before the wound dressing becomes blocked.

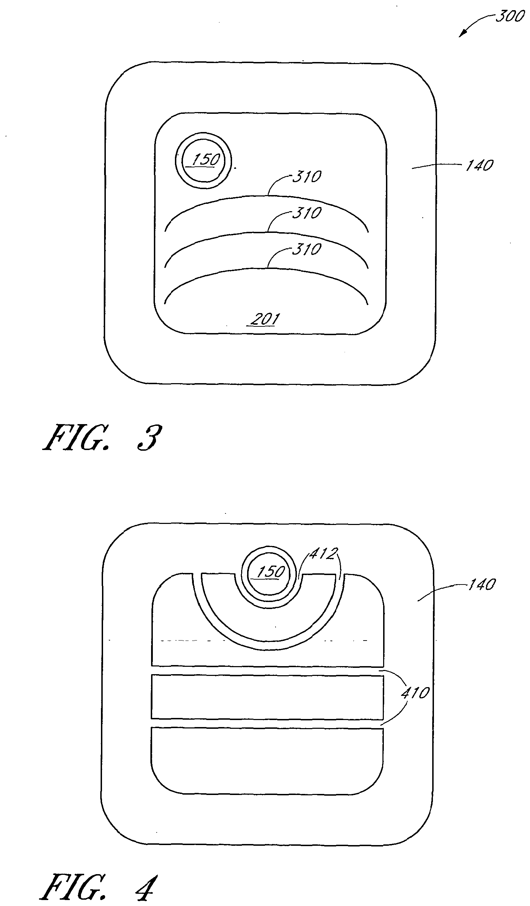

Figure 3 shows a plan view of a wound dressing including baffle elements that reduce the rate at which wound exudate moves towards the orifice according to one embodiment of the invention. The wound dressing illustrated in Figure 3 is similar to that shown in Figures 1 and 2, but includes a number of baffle elements 310 disposed across the central raised region 201. The baffle elements 310 form barriers in the central region of the dressing, which arrest the movement of wound exudate towards the orifice.

Embodiments of baffle elements that may be used in the wound dressing described herein are preferably at least partly flexible, so as to permit the wound dressing to flex and conform with the skin of the patient surrounding the wound site. When so present in the wound dressing, the baffle elements are preferably constructed so as to at least partially prevent liquid from flowing directly to the wound dressing port or orifice and its associated filter, if so provided. The baffle elements thus increase the distance that liquids may require to reach the port, which may help in absorbing these fluids into the absorbent or superabsorbent material of the wound dressing.

According to some embodiments of the invention, the baffle element may comprise a sealing region in which the absorbent layer 110 and transmission layer 105 are absent and cover layer 140 is sealed to the wound contact layer 101. Thus, the baffle element presents a barrier to the motion of the wound exudate, which must therefore follow a path that avoids the baffle element. Thus the time taken for the wound exudate to reach the orifice is increased.

In some embodiments, the baffle elements may be an insert of a substantially nonporous material, for example a closed-cell polyethylene foam, placed inside the dressing. In some cases, it may be preferable to place such an inserted baffle element in a sealing region where one or more of the absorbent layer 110 and/or transmission layer 105 are absent. A sealant, for example a viscous curing sealant such as a silicone sealant, could be placed or injected as a thin strip so as to form a baffle element that is substantially liquid impermeable. Such a baffle element could be placed or injected into a region of the transmission layer 105 and/or absorbent layer 110, or also a sealing region where the absorbent layer 110 and/or transmission layer 105 are absent.

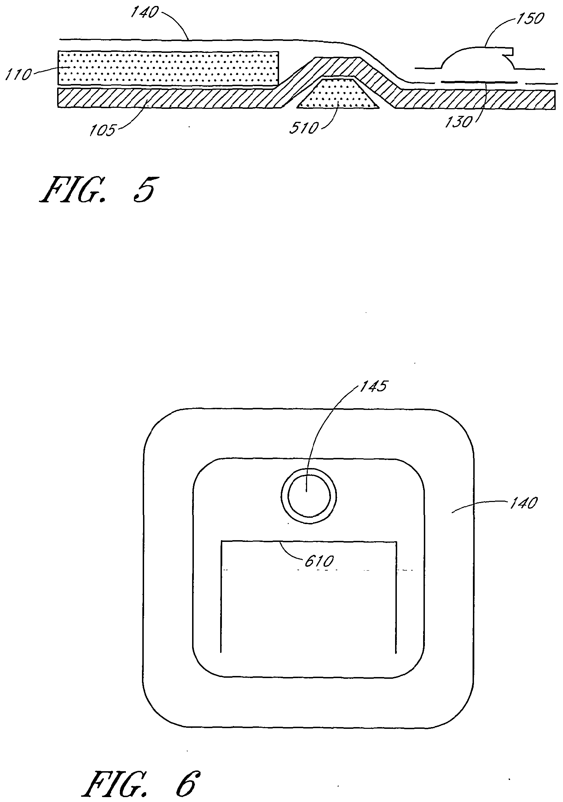

Figure 6 illustrates a wound dressing including a baffle element according to a further embodiment of the invention. A single baffle element 610 provides a cup shaped barrier between the bulk of the absorbent layer 110 and the orifice 145. Thus wound exudate that is initially drawn from the wound site within the region defined by the baffle element 610, must follow a path around the outside of the cup shaped barrier to reach the orifice 145. As will be recognized, the baffle element 610 reduces the effect of gravity on reducing the time taken for the wound exudate to move to the orifice 145, as for most orientations of the wound dressing at least a part of the path taken by the wound exudate will be against the force of gravity.

The embodiments of Figures 3 and 6 have been described with respect to a wound dressing having a structure as shown in Figure 1. However, it will be understood that the baffle elements could equally be applied to a wound dressing in which the transmission layer 105 was absent.

Figure 4 shows a plan view of a wound dressing including the at least one element according to once embodiment of the invention in which a number of baffle elements 410 are provided that extend across the width of the central region 201 of the wound dressing, with further baffle elements 412 formed in a semi-circular path around the orifice 145.

Figure 5 illustrates the configuration of baffle elements 410 according to some embodiments of the invention. The baffle element comprises a channel of absorbent material 510 underlying the transmission layer 105. A channel in the absorbent layer 110 is located over the baffle element 410 so that the transmission layer is in contact with the cover layer 140 in the region of the baffle element 410. Thus, wound exudate that is moving along a lower surface of the transmission layer 105, and has therefore not been drawn into absorbent layer 110, will come into contact with and be absorbed by the channel of absorbent material 510.

Alternatively, or additionally, baffle elements may comprise one or more channels provided in the surface of the transmission layer 105 underlying and abutting the absorbent layer 110. In use, when negative pressure is applied to the wound dressing, the absorbent layer 110 will be drawn into the channel. The channel in the transmission layer may have a depth substantially equal to the depth of the transmission layer, or may have a depth less than the depth of the transmission layer. The dimensions of the channel may be chosen to ensure that the channel is filled by the absorbent layer 110 when negative pressure is applied to the wound dressing. According to some embodiments, the channel in the transmission layer comprises a channel of absorbent material in the transmission layer 105.

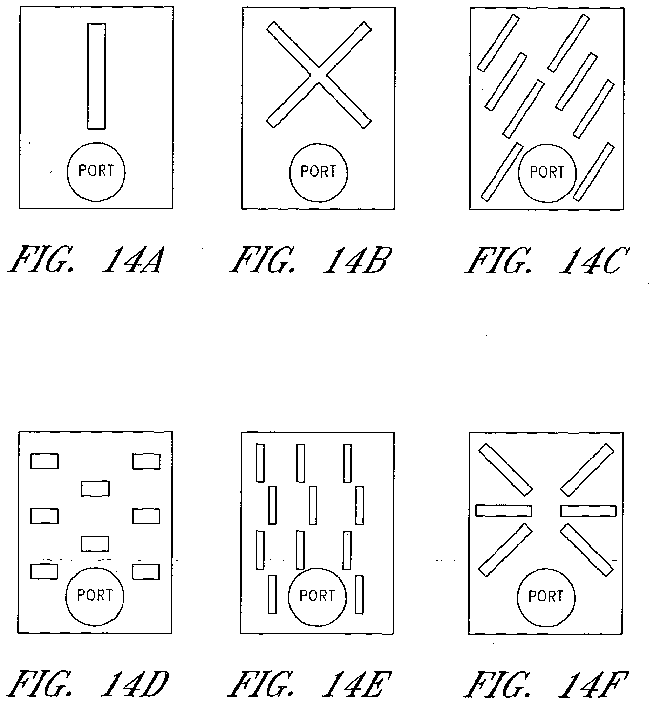

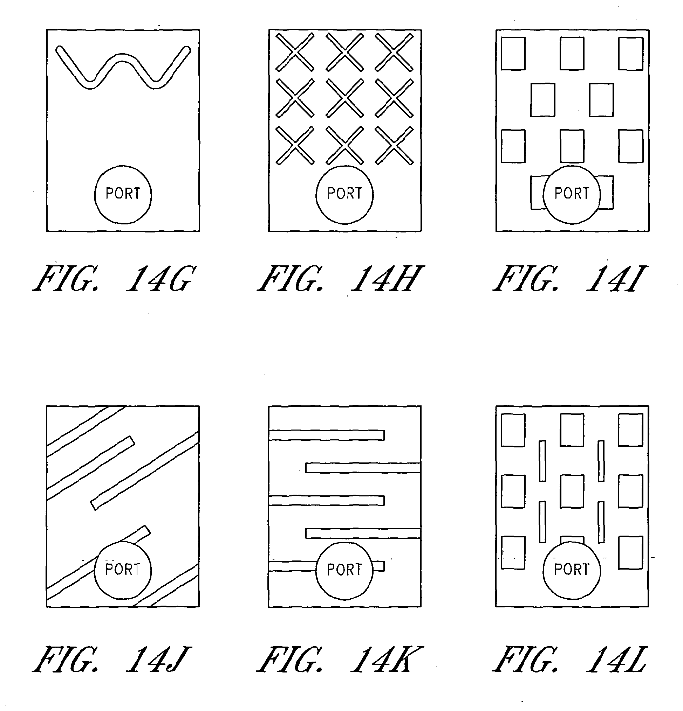

The baffle elements may be formed into a range of shapes and patterns, for example Figures 14A to 14L illustrate wound dressings having a number of different exemplifying configurations of baffle elements. Figure 14A illustrates a linear baffle element in a vertical configuration aligned in the direction of the port or orifice. Figure 14B illustrates an X-shaped baffle element. Figures 14C-E illustrate embodiments of wound dressings with multiple baffle elements, aligned in a generally diagonal, horizontal, or vertical manner.

Figure 14F illustrates baffle elements arranged in a six-armed starburst configuration, with a center portion left open. Figure 14G illustrates a W-shaped baffle element on the wound dressing in a position distal to the port or orifice. In Figure 14H, an 3-by-3 array of X-shaped baffle elements is provided on the wound dressing, although it will be understood that more or less X-shaped baffle elements may be used. Figure 14I shows an embodiment with a plurality of rectangular baffle elements, and wherein one or more baffle elements are located underneath the port in the wound dressing. Figures 14J-K illustrate wound dressing embodiments with longer diagonal and horizontal baffle elements. In Figure 14L, rectangular baffle elements are present on this embodiment of a wound dressing, wherein the baffle elements are of different sizes.

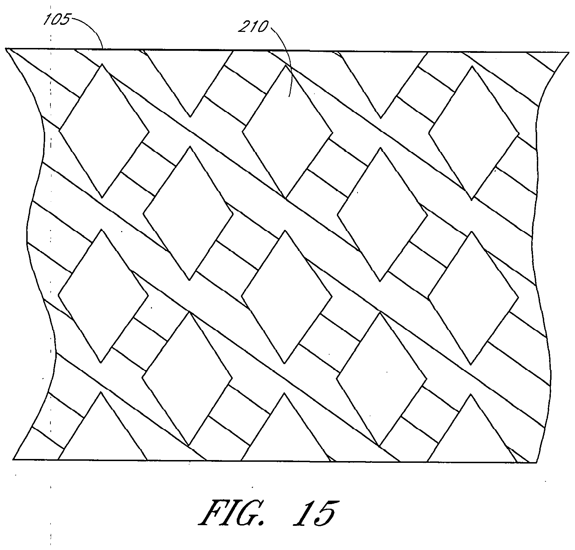

According to some embodiments of the invention, the at least one element comprises an array of vias, or troughs, in the transmission layer 105. Figure 15 illustrates a transmission layer 105 that is perforated with diamond shaped vias 210. The vias 210 are arranged such that no linear pathway exists through the pattern of vias that does not intersect with one or more of the vias 210.

When negative pressure is applied to the wound dressing, the absorbent layer 110 is drawn into the vias 210, increasing the area of the absorbent layer that comes into contact with wound exudate being drawn through the transmission layer 105. Alternatively, the vias 210 may be filled with further absorbent material for absorbing wound exudate being drawn through the transmission layer 105. The vias may extend through the depth of the transmission layer 105, or may extend through only part of the transmission layer.

Wound exudate moving through the transmission layer 105 under the influence of gravity will fall through the transmission layer in a substantially linear manner. Any such linear pathways will, at some point, intersect with one of the vias 210, and thus the exudate will be brought into contact with absorbent material within the vias 210. Wound exudate coming into contact with absorbent material will be absorbed, stopping the flow of the wound exudate through the transmission layer 105, and reducing the amount of unabsorbed wound exudate that may otherwise pool around the orifice. It will be appreciated that the vias are not limited to diamond shapes, and that any pattern of vias may be used. Preferably, the vias will be arranged to ensure that all linear paths through the transmission layer 105 intersect with at least one via. The pattern of vias may be chosen to minimise the distance that wound exudate is able to travel though the transmission layer before encountering a via and being absorbed.

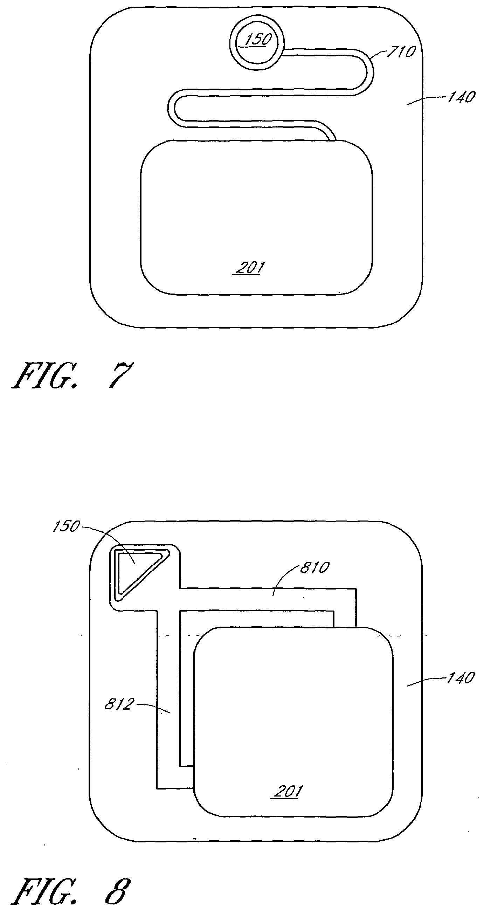

Figure 7 illustrates a wound dressing in accordance with some embodiments of the invention in which the at least one element comprises an air channel 710 connecting the central region 201 of the wound dressing to the orifice 145. In the embodiment of Figure 7, the air channel 710 extends from an edge region of the transmission layer 105 and connects the transmission layer to the orifice 145.

In use, wound exudate is drawn towards the orifice 145 by the application of negative pressure at the suction port 150. However, the air channel 710 present a relatively long serpentine path to be followed by the wound exudate before it reaches the orifice 145. This long path increases the time that negative pressure can be applied to the dressing before wound exudate traverses the distance between the transmission layer and the orifice and blocks the filter element 130, thereby increasing the time the dressing can be in use before it must be replaced.

Figure 8 illustrates a wound dressing in accordance with one embodiment of the invention in which the at least one element comprises air channels 810 and 812 connecting the central region 201 of the wound dressing to the orifice 145. Channels 810 and 812 are coupled to the transmission layer at substantially opposite corners of the central region 201.

The wound dressing shown in Figure 8 reduces the effect of gravity on the time taken for the orifice to become blocked. If the wound dressing is in an orientation in which wound exudate moves under the influence of gravity towards the edge region of the transmission layer connected to air channel 810, the effect of gravity will be to move wound exudate away from the edge region of the transmission layer coupled to air channel 812, and vice versa. Thus, the embodiment of Figure 8 provides alternative air channels for coupling the negative pressure to the transmission layer such that, should one air channel become blocked a remaining air channel should remain open and able to communicate the negative pressure to the transmission layer 105, thereby increasing the time before negative pressure can no longer be applied to the wound dressing and the dressing must be changed.

Further embodiments of the invention may comprise greater numbers of air channels connecting the transmission layer 105 to the orifice.

According to some embodiments of the invention, two or more orifices may be provided in the cover layer 140 for applying the negative pressure to the wound dressing. The two or more orifices can be distributed across the cover layer 140 such that if one orifice becomes blocked by wound exudate due to the wound dressing being in a particular orientation, at least one remaining orifice would be expected to remain unblocked. Each orifice is in fluid communication with a wound chamber defined by the wound dressing, and is therefore able to communicate the negative pressure to the wound site.

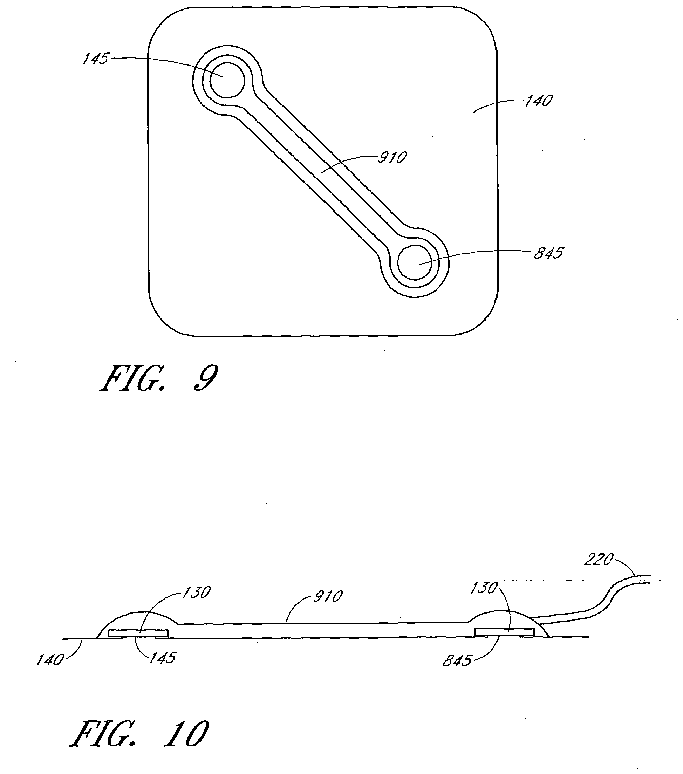

Figure 9 illustrates a wound dressing in accordance with a further embodiment of the invention. The wound dressing of figure 9 is similar to that of Figure 1 but includes two orifices 145 and 845 provided in the cover layer 140. A fluid communication passage connects the two orifices such that a negative pressure applied to one of the orifices is communicated to the remaining orifice via the fluid communication passage. The orifices 145, 845 are located in opposite corner regions of the cover layer 140. The fluid communication passage is formed using a flexible moulding 910 on the upper surface of the cover layer 140. It will be appreciated that the flexible moulding may be formed from other suitable means for example a strip of transmission or open porous foam layer placed on the cover layer 140 between the orifices 145 and 845 and a further film welded or adhered over the strip thus sealing it to the cover layer and forming a passageway through the foam. A conduit may then be attached in a known manner to the sealing film for application of negative pressure.

In use, the wound dressing having two orifices is sealed over a wound site to form a wound cavity and an external source of negative pressure is applied to one of the orifices 145, 845, and the negative pressure will be communicated to the remaining orifice via the fluid communication passage. Thus, the negative pressure is communicated via the two orifices 145, 845 to the transmission layer 105, and thereby to the wound site. If one of the orifices 145, 845 becomes blocked due to wound exudate collecting at the orifice under the influence of gravity, the remaining orifice should remain clear, allowing negative pressure to continue to be communicated to the wound site. According to some embodiments, the transmission layer 105 may be omitted, and the two orifices will communicate the negative pressure to the wound site via the absorbent layer 110.

Figure 10 illustrates a side view of the fluid communication passage of the embodiment of figure 9. Moulding 910 is sealed to the top surface of the cover layer 140, and covering orifices 145 and 845. Gas permeable liquid impermeable filter elements 130 are provided at each orifice. The moulding 910 is coupled to an external source of negative pressure via a tube element 220.

According to some embodiments, a single filter element may be used extending underneath the length of the fluid communication passage and the two orifices. While the above example embodiment has been described as having two orifices, it will be understood that more than two orifices could be used, the fluid communication passage allowing the negative pressure to be communicated between the orifices.

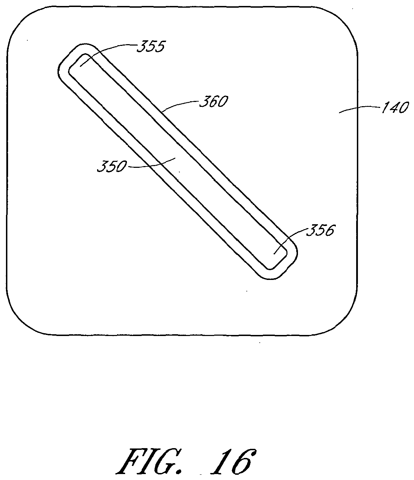

Figure 16 illustrates an alternative arrangement in which a single elongate orifice 350 is provided in the cover layer 140. First and second ends 355, 356 of the orifice 350 are located in opposite corner regions of the cover layer 140. A flexible molding 360 is sealed around the orifice 350 and allows negative pressure to be communicated through the cover layer 140 along the length of the orifice 350. The flexible moulding 360 may be formed by any suitable means as described above in relation to flexible moulding 910.

In use, the wound dressing is sealed over a wound site to form a wound cavity and an external source of negative pressure is applied to the orifice. If, due to the orientation of the wound dressing, wound exudate moves under the influence of gravity to collect around one end 355 of the orifice 350, a portion of the orifice 350 near to the end 355 will become blocked. However, a portion of the orifice near to the remaining end 356 should remain clear, allowing continued application of negative pressure to the wound site.

As still further options the dressing can contain anti-microbial e.g. nanocrystalline silver agents on the wound contact layer and/or silver sulphur diazine in the absorbent layer. These may be used separately or together. These respectively kill micro-organisms in the wound and micro-organisms in the absorption matrix. As a still further option other active components, for example, pain suppressants, such as ibuprofen, may be included. Also agents which enhance cell activity, such as growth factors or that inhibit enzymes, such as matrix metalloproteinase inhibitors, such as tissue inhibitors of metalloproteinase (TIMPS) or zinc chelators could be utilised. As a still further option odour trapping elements such as activated carbon, cyclodextrine, zealite or the like may be included in the absorbent layer or as a still further layer above the filter layer.

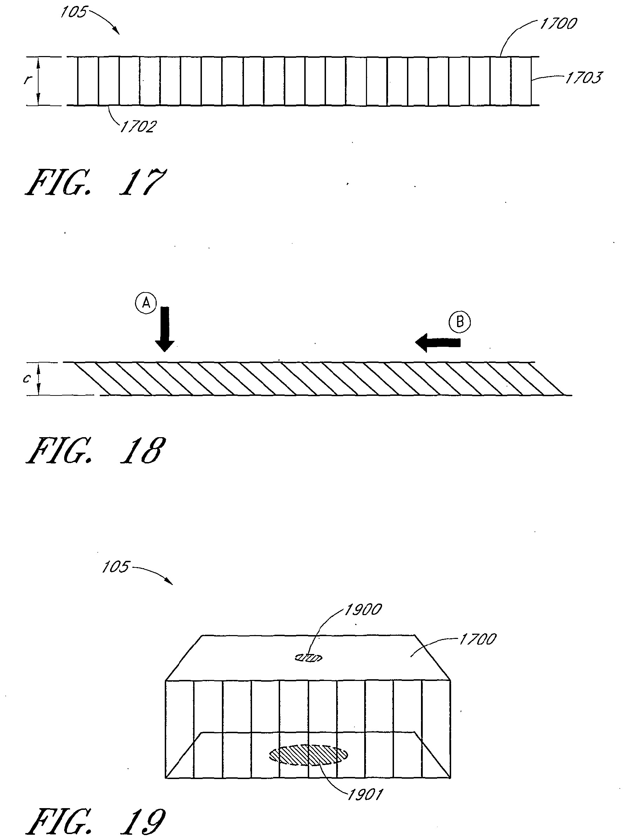

Figure 17 illustrates a first, upper surface 1700 and a further, lower surface 1702 of a transmission layer 105 according to an embodiment of the present invention. In the embodiment illustrated in Figure 17 fibres 1703 of a woven layer extend between the first surface 1700 and the further surface 1702. It will be appreciated that according to further embodiments of the present invention if a foam layer is used as a transmission layer 105 the connected strands forming the foam will act as spacer elements. As illustrated in Figure 17 in a relaxed mode of operation, that is to say when in use, no negative pressure is applied to the wound dressing or negative pressure is applied to the wound dressing but no external force acts on the wound dressing then the fibres 1703 extend substantially perpendicular to the upper and lower surfaces keeping the surfaces in a spaced apart substantially parallel configuration.

Figure 18 illustrates the transmission layer 105 when an external force is exerted on the outside of the dressing. The external force can be a compressive force indicated by arrow A and/or a lateral force illustrated by arrow B in Figure 18. As indicated either a compressive force or a lateral force acts to cause the fibres 1703 to lean to one side. This causes the upper and lower surfaces to become laterally offset with respect to each other as well as causing the thickness of the layer to reduce from a separation distance r indicated in Figure 17 in a relaxed mode of operation to a compression distance c illustrated in Figure 18. The reduction in thickness effectively provides some "give" in the dressing even when the dressing is subject to negative pressure. It will be appreciated that the forces acting on the dressing may occur throughout the whole of the surface area of the dressing or only in one or more particular regions. In such a situation regions of the dressing can be in a relaxed mode of operation and further regions can be in a compressed mode of operation. As illustrated in Figure 18 when a force is exerted on the transmission layer the fibres separating the upper and lower surfaces tend to lean to one side sharing a common lean angle.

Throughout this specification reference will be made to a relaxed mode of operation and a forced mode of operation. It is to be understood that the relaxed mode of operation corresponds to a natural state of the material either when no negative pressure is applied or when negative pressure is applied. In either situation no external force, caused for example by motion of a patient or an impact is in evidence. By contrast a forced mode of operation occurs when an external force whether compressive, lateral or other is brought to bear upon the wound dressing. Such forces can cause serious damage/prevent healing or a wound.

Figure 19 illustrates how certain embodiments of the present invention can also operate to offset load forces. As illustrated in Figure 19 if a force is exerted over a contact area 1900 in an upper surface 1700 of the transmission layer 105 then this force is transmitted across and through the transmission layer and is exerted over a larger dissipation area 1901 against an underlying wound site. In the case of use of a 3D knit as a transmission layer this is because the relatively stiff spacer elements provide at least some lateral stiffness to the layer.

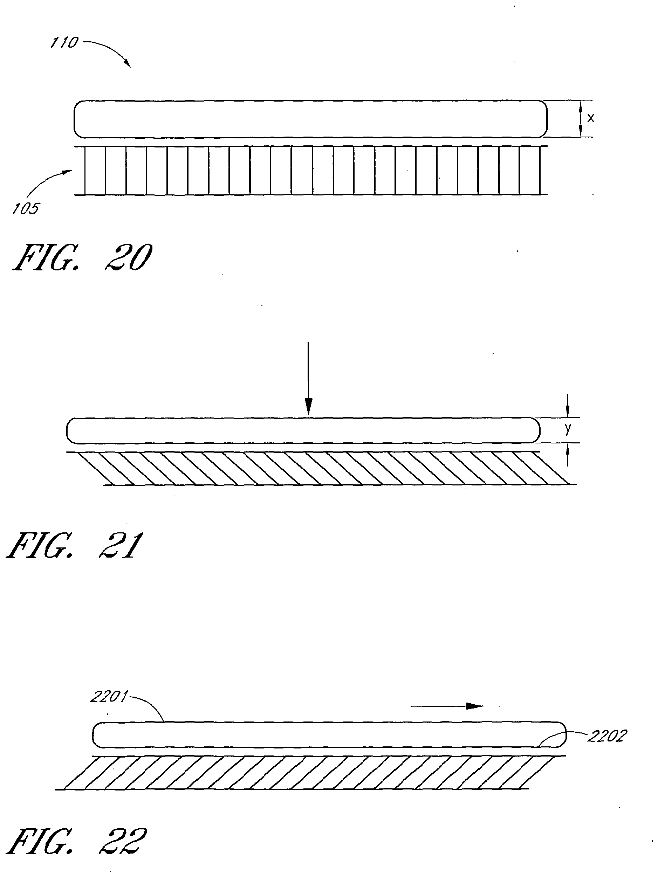

Figure 20 illustrates the transmission layer 105 and absorbent layer 110 in more detail. The absorbent layer 110 is located proximate to the upper surface 1700 of the transmission layer 105 and is unbonded thereto according to certain embodiments of the present invention. When unbonded the absorbent layer 110 is also able to move laterally with respect to the underlying transmission layer when a lateral or shear force is applied to the wound dressing. Also the absorbent layer is able to further compress when a compressive force illustrated in Figure 21 acts on the wound dressing. As illustrated in Figure 21 the absorbent layer 110 decreases in thickness under a compressive force from a non-compressed thickness x illustrated in Figure 20 to a compressed distance y illustrated in Figure 21. The compressive force also acts to offset the upper and lower surfaces of the transmission layer as described above thus enhancing the "give" of the dressing. The ability for an upper surface 2201 to translate laterally with respect to a lower surface 2202 of the absorbent layer under a lateral or shearing force exerted on the wound dressing is illustrated in more detail in Figure 22. This lateral motion causes the thickness x of the absorbent layer 110 to reduce and the upper surface and lower surface of the absorbent layer to be offset with respect to each other. This effect can itself be sufficient to prevent shear forces exerted on the whole or part of the wound dressing from being transferred to an underlying wound bed. As can the corresponding effect in the transmission layer. However a combination enhances the cushioning effect. If the wound bed comprises a skin graft region the reduction of shear forces can be particularly advantageous.

Figure 23 illustrates an embodiment of a TNP wound treatment comprising a wound dressing 100 in combination with a pump 800. Here, the dressing 100 may be placed over a wound as described previously, and a conduit 220 may then be connected to the port 150, although in some embodiments the dressing 100 may be provided with at least a portion of the conduit 220 preattached to the port 150. Preferably, the dressing 100 is provided as a single article with all wound dressing elements (including the port 150) preattached and integrated into a single unit. The wound dressing 100 may then be connected, via the conduit 220, to a source of negative pressure such as the pump 800. Preferably, the pump 800 is miniaturized and portable, although larger conventional pumps may also be used with the dressing 100. In some embodiments, the pump 800 may be attached or mounted onto or adjacent the dressing 100. A connector 221 may also be provided so as to permit the conduit 220 leading to the wound dressing 100 to be disconnected from the pump, which may be useful for example during dressing changes.

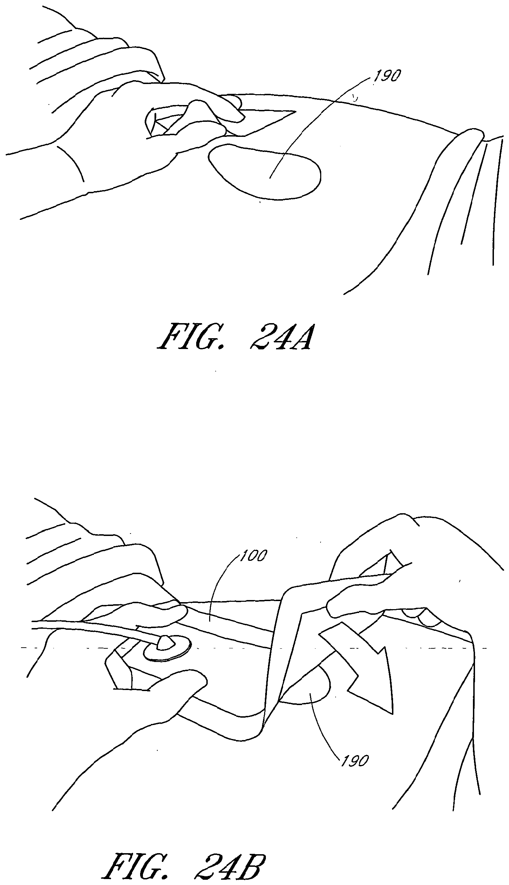

Figures 24A-D illustrate the use of an embodiment of a TNP wound treatment system being used to treat a wound site on a patient. Figure 24A shows a wound site 190 being cleaned and prepared for treatment. Here, the healthy skin surrounding the wound site 190 is preferably cleaned and excess hair removed or shaved. The wound site 190 may also be irrigated with sterile saline solution if necessary. Optionally, a skin protectant may be applied to the skin surrounding the wound site 190. If necessary, a wound packing material, such as foam or gauze, may be placed in the wound site 190. This may be preferable if the wound site 190 is a deeper wound.

After the skin surrounding the wound site 190 is dry, and with reference now to Figure 24B, the wound dressing 100 may be positioned and placed over the wound site 190. Preferably, the wound dressing 100 is placed with the wound contact layer 102 over and/or in contact with the wound site 190. In some embodiments, an adhesive layer is provided on the lower surface 101 of the wound contact layer 102, which may in some cases be protected by an optional release layer to be removed prior to placement of the wound dressing 100 over the wound site 190. Preferably, the dressing 100 is positioned such that the port 150 is in a raised position with respect to the remainder of the dressing 100 so as to avoid fluid pooling around the port. In some embodiments, the dressing 100 is positioned so that the port 150 is not directly overlying the wound, and is level with or at a higher point than the wound. To help ensure adequate sealing for TNP, the edges of the dressing 100 are preferably smoothed over to avoid creases or folds.

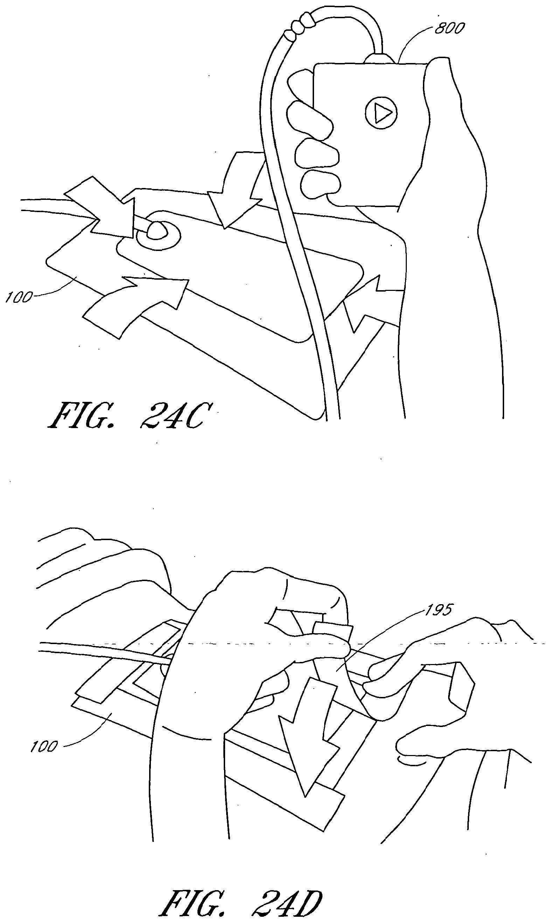

With reference now to Figure 24C, the dressing 100 is connected to the pump 800. The pump 800 is configured to apply negative pressure to the wound site via the dressing 100, and typically through a conduit. In some embodiments, and as described above in Figure 23, a connector may be used to join the conduit from the dressing 100 to the pump 800. Upon the application of negative pressure with the pump 800, the dressing 100 may in some embodiments partially collapse and present a wrinkled appearance as a result of the evacuation of some or all of the air underneath the dressing 100. In some embodiments, the pump 800 may be configured to detect if any leaks are present in the dressing 100, such as at the interface between the dressing 100 and the skin surrounding the wound site 190. Should a leak be found, such leak is preferably remedied prior to continuing treatment.

Turning to Figure 24D, additional fixation strips 195 may also be attached around the edges of the dressing 100. Such fixation strips 195 may be advantageous in some situations so as to provide additional sealing against the skin of the patient surrounding the wound site 190. For example, the fixation strips 195 may provide additional sealing for when a patient is more mobile. In some cases, the fixation strips 195 may be used prior to activation of the pump 800, particularly if the dressing 100 is placed over a difficult to reach or contoured area.

Treatment of the wound site 190 preferably continues until the wound has reached a desired level of healing. In some embodiments, it may be desirable to replace the dressing 100 after a certain time period has elapsed, or if the dressing is full of wound fluids. During such changes, the pump 800 may be kept, with just the dressing 100 being changed.

It is to be noted that in use the dressing may be used "up-side down", at an angle or vertical. References to upper and lower are thus used for explanation purposes only.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A wound dressing for providing protection at a wound site, comprising:
a transmission layer (105) comprising a first, upper surface and a further lower surface spaced apart from the first surface by a relax distance in a relaxed mode of operation, wherein the first, upper surface comprises a top spacer fabric layer and the further, lower surface comprises a bottom spacer fabric layer, **characterised in that** the filament count in the top layer is greater than the filament count in the bottom layer so that liquid tends to be wicked along the top layer more than the bottom layer;
a plurality of spacer elements extending between the first and further surfaces and, in a forced mode of operation, locatable whereby the first and further surfaces are spaced apart by a compression distance less than the relax distance; and
an absorbent layer (110) for absorbing wound exudate, the absorbent layer overlying the transmission layer.

2. The wound dressing (100) as claimed in claim 1, further comprising:
each spacer element comprises a fibre element extending between and substantially perpendicular to the first and further surfaces in the relaxed mode of operation.

3. The wound dressing (100) as claimed in claim 1 or claim 2, further comprising:
in the forced mode of operation, the first and further surfaces are laterally offset with respect to each other.

4. The wound dressing (100) as claimed in claim 2, further comprising:
in the forced mode of operation, each fibre element is at least partially lent aside away from the perpendicular position with respect to the first and further surfaces.

5. The wound dressing (100) as claimed in claim 4, further comprising:
each fibre in a region of the transmission layer (105) in a forced mode of operation, is lent in a substantially common direction with a substantially common lean angle.

6. The wound dressing (100) as claimed in claim 1, further comprising:
the absorbent layer (110) comprises a pad of non-woven material permitting compression and lateral translation of respective regions of the pad.

7. The wound dressing (100) as claimed in claim 1 or 6 further comprising the pad is a foam or non-woven natural or synthetic material optionally comprising superabsorber material.

8. The wound dressing (100) as claimed in any preceding claim wherein the transmission layer comprises a knitted layer of at least partially elastomeric material.

9. The wound dressing (100) as claimed in claim 8, wherein the elastomeric material is a polyester material.

10. The wound dressing (100) as claimed in any preceding claim, further comprising:
the first and further surfaces and spacer elements are disposed to dissipate force from a first region comprising a contact area of the first surface to a further region comprising a dissipation area, greater than the contact area, of the further surface.

11. The wound dressing (100) as claimed in claim 10 wherein the material of the spacer elements has a lateral stiffness sufficient to dissipate force.

12. The wound dressing (100) as claimed in any preceding claim, further comprising:
a moisture vapour permeable drape layer (140) over the transmission layer; and
a suction port (150) connectable to a source of negative pressure.

13. The wound dressing (100) as claimed in any preceding claim, further comprising:
the force is a compressive force acting substantially perpendicular to the first and further surfaces and/or a lateral force acting substantially parallel to the first and further surfaces so as to create a shear force between the first and further surfaces.

14. The wound dressing (100) of claim 12, wherein the suction port (150) further comprises:
a sealing surface (152) for sealing the suction port (150) to the wound dressing (100);
a connector portion (154) for connecting the suction port (150) to a source of negative pressure, and
a hemispherical body portion (156) disposed between the sealing surface (152) and the connector portion (154);
the sealing surface (152) comprising a flange that provides a flat area to provide a good seal when the port (150) is sealed to the cover layer (140).

15. The wound dressing (100) as claimed in any previous claim, wherein the transmission layer (105) is smaller than the absorbent layer (110).

16. The wound dressing (100) as claimed in any previous claim, further comprising:
under a compressive force the absorbent layer (105) decreases in thickness.

17. The wound dressing (100) as claimed in claims 1 through 16, further comprising:
under compressive force, the absorbent layer (110) decreases in thickness and the compressive force acts to offset the first and further surfaces of the transmission layer (105).

## Patentansprüche

1. Ein Wundverband zum Bereitstellen von Schutz an einer Wundstelle, beinhaltend: eine Übertragungsschicht (105), beinhaltend eine erste, obere Oberfläche und eine weitere, untere Oberfläche, die in einer Entspannungsfunktionsweise von der ersten Oberfläche um einen Entspannungsabstand beabstandet ist, wobei die erste, obere Oberfläche eine Abstandshalter-Stoffoberschicht und die weitere, untere Oberfläche eine Abstandshalter-Stoffunterschicht beinhaltet, **dadurch gekennzeichnet, dass** die Filamentzahl in der Oberschicht größer ist als die Filamentzahl in der Unterschicht, sodass Flüssigkeit dazu neigt, durch Dochtwirkung eher entlang der Oberschicht als der Unterschicht gesaugt zu werden;
eine Vielzahl von Abstandhalterelementen, die sich zwischen der ersten und der weiteren Oberfläche erstrecken und in einer Zwangsfunktionsweise derart positionierbar sind, dass die erste und die weitere Oberfläche um einen Kompressionsabstand, der kleiner als der Entspannungsabstand ist, voneinander beabstandet sind; und
eine absorbierende Schicht (110) zum Absorbieren von Wundexsudat, wobei die absorbierende Schicht über der Übertragungsschicht liegt.

2. Wundverband (100) gemäß Anspruch 1, ferner beinhaltend:
Jedes Abstandshalterelement beinhaltet ein Faserelement, das sich in der Entspannungsfunktionsweise zwischen und im Wesentlichen senkrecht zu der ersten und der weiteren Oberfläche erstreckt.

3. Wundverband (100) gemäß Anspruch 1 oder Anspruch 2, ferner beinhaltend:
In der Zwangsfunktionsweise sind die erste und die weitere Oberfläche in Bezug aufeinander seitlich versetzt.

4. Wundverband (100) gemäß Anspruch 2, ferner beinhaltend:
In der Zwangsfunktionsweise ist jedes Faserelement zumindest teilweise in Bezug auf die erste und die weitere Oberfläche von der senkrechten Stellung weg geneigt.

5. Wundverband (100) gemäß Anspruch 4, ferner beinhaltend:
Jede Faser in einer Region der Übertragungsschicht (105) ist in einer Zwangsfunktionsweise in einer im Wesentlichen gemeinsamen Richtung unter einem im Wesentlichen gemeinsamen Neigungswinkel geneigt.

6. Wundverband (100) gemäß Anspruch 1, ferner beinhaltend:
Die absorbierende Schicht (110) beinhaltet ein Kissen aus Vliesmaterial, was eine Kompression und eine seitliche Translation der jeweiligen Regionen des Kissens ermöglicht.

7. Wundverband (100) gemäß Anspruch 1 oder 6, ferner beinhaltend, dass das Kissen ein natürliches oder synthetisches Schaumstoff- oder Vliesmaterial ist, welches optional Superabsorbermaterial beinhaltet.

8. Wundverband (100) gemäß einem der vorhergehenden Ansprüche, wobei die Übertragungsschicht eine gestrickte Schicht aus mindestens teilweise elastomerem Material beinhaltet.

9. Wundverband (100) gemäß Anspruch 8, wobei das elastomere Material ein Polyestermaterial ist.

10. Wundverband (100) gemäß einem der vorhergehenden Ansprüche, ferner beinhaltend: Die erste und die weitere Oberfläche und die Abstandshalterelemente sind angeordnet, um Kraft von einer ersten Region, die einen Kontaktbereich der ersten Oberfläche beinhaltet, auf eine weitere Region, beinhaltend einen Dissipationsbereich, der größer als der Kontaktbereich ist, der weiteren Oberfläche zu verteilen.

11. Wundverband (100) gemäß Anspruch 10, wobei das Material der Abstandshalterelemente eine seitliche Steifigkeit aufweist, die ausreicht, um Kraft zu verteilen.

12. Wundverband (100) gemäß einem der vorhergehenden Ansprüche, ferner beinhaltend: eine feuchtigkeitsdampfdurchlässige Abdeckschicht (140) über der Übertragungsschicht; und
einen Sauganschluss (150), der mit einer Unterdruckquelle verbunden werden kann.

13. Wundverband (100) gemäß einem der vorhergehenden Ansprüche, ferner beinhaltend: Die Kraft ist eine im Wesentlichen senkrecht zu der ersten und der weiteren Oberfläche wirksame Kompressionskraft und/oder eine im Wesentlichen parallel zu der ersten und der weiteren Oberfläche wirkende seitliche Kraft, um eine Scherkraft zwischen der ersten und der weiteren Oberfläche zu erzeugen.

14. Wundverband (100) gemäß Anspruch 12, wobei der Sauganschluss (150) ferner Folgendes beinhaltet:
eine Dichtfläche (152) zum Abdichten des Sauganschlusses (150) gegenüber dem Wundverband (100);
einen Verbinderabschnitt (154) zum Verbinden des Sauganschlusses (150) mit einer Unterdruckquelle und
einen halbkugelförmigen Körperabschnitt (156), der zwischen der Dichtfläche (152) und dem Verbinderabschnitt (154) angeordnet ist;
wobei die Dichtfläche (152) einen Flansch beinhaltet, der einen ebenen Bereich bereitstellt, um eine gute Abdichtung bereitzustellen, wenn der Anschluss (150) gegenüber der Deckschicht (140) abgedichtet ist.

15. Wundverband (100) gemäß einem der vorhergehenden Ansprüche, wobei die Übertragungsschicht (105) kleiner als die absorbierende Schicht (110) ist.

16. Wundverband (100) gemäß einem der vorhergehenden Ansprüche, ferner beinhaltend: Unter einer Kompressionskraft nimmt die absorbierende Schicht (105) an Dicke ab.

17. Wundverband (100) gemäß einem der Ansprüche 1 bis 16, ferner beinhaltend:
Unter Kompressionskraft nimmt die absorbierende Schicht (110) an Dicke ab und die Kompressionskraft wirkt, um die erste und die weitere Oberfläche der Übertragungsschicht (105) zu versetzen.

## Revendications

1. Un pansement pour plaie destiné à fournir une protection au niveau d'un site de plaie, comprenant :
une couche de transmission (105) comprenant une première surface supérieure et une surface supplémentaire inférieure espacée de la première surface d'une distance de détente dans un mode de fonctionnement détendu, dans lequel la première surface supérieure comprend une couche de tissu d'espacement de dessus et la surface supplémentaire inférieure comprend une couche de tissu d'espacement de dessous, **caractérisé en ce que** le compte de filaments dans la couche de dessus est supérieur au compte de filaments dans la couche de dessous de sorte qu'un liquide a tendance à drainer par effet de mèche le long de la couche de dessus davantage que le long de la couche de dessous ;
une pluralité d'éléments d'espacement s'étendant entre la première surface et la surface supplémentaire et, dans un mode de fonctionnement forcé, pouvant être localisés moyennant quoi la première surface et la surface supplémentaire sont espacées d'une distance de compression inférieure à la distance de détente ; et
une couche absorbante (110) destinée à absorber un exsudat de plaie, la couche absorbante recouvrant la couche de transmission.

2. Le pansement pour plaie (100) tel que revendiqué dans la revendication 1, comprenant en outre :
chaque élément d'espacement comprend un élément de fibre s'étendant entre et substantiellement perpendiculaire à la première surface et la surface supplémentaire dans le mode de fonctionnement détendu.

3. Le pansement pour plaie (100) tel que revendiqué dans la revendication 1 ou la revendication 2, comprenant en outre :
dans le mode de fonctionnement forcé, la première surface et la surface supplémentaire sont décalées latéralement l'une par rapport à l'autre.

4. Le pansement pour plaie (100) tel que revendiqué dans la revendication 2, comprenant en outre :
dans le mode de fonctionnement forcé, chaque élément de fibre est au moins partiellement penché en l'éloignant de la position perpendiculaire par rapport à la première surface et la surface supplémentaire.

5. Le pansement pour plaie (100) tel que revendiqué dans la revendication 4, comprenant en outre :
chaque fibre dans une région de la couche de transmission (105) dans un mode de fonctionnement forcé, est penchée dans une direction substantiellement commune selon un angle oblique substantiellement commun.

6. Le pansement pour plaie (100) tel que revendiqué dans la revendication 1, comprenant en outre :
la couche absorbante (110) comprend un coussinet de matériau non tissé permettant la compression et la translation latérale de régions respectives du coussinet.

7. Le pansement pour plaie (100) tel que revendiqué dans la revendication 1 ou la revendication 6 comprenant en outre le fait que le coussinet est un matériau naturel ou synthétique non tissé ou en mousse comprenant facultativement un matériau superabsorbant.

8. Le pansement pour plaie (100) tel que revendiqué dans n'importe quelle revendication précédente dans lequel la couche de transmission comprend une couche tricotée de matériau au moins partiellement élastomère.

9. Le pansement pour plaie (100) tel que revendiqué dans la revendication 8, dans lequel le matériau élastomère est un matériau polyester.

10. Le pansement pour plaie (100) tel que revendiqué dans n'importe quelle revendication précédente, comprenant en outre :
la première surface et la surface supplémentaire et les éléments d'espacement sont disposés pour dissiper une force d'une première région comprenant une zone de contact de la première surface à une région supplémentaire comprenant une zone de dissipation, supérieure à la zone de contact, de la surface supplémentaire.

11. Le pansement pour plaie (100) tel que revendiqué dans la revendication 10 dans lequel le matériau des éléments d'espacement a une rigidité latérale suffisante pour dissiper une force.

12. Le pansement pour plaie (100) tel que revendiqué dans n'importe quelle revendication précédente, comprenant en outre : une couche de champ perméable à la vapeur d'eau (140) par-dessus la couche de transmission ; et
un orifice d'aspiration (150) pouvant être raccordé à une source de pression négative.

13. Le pansement pour plaie (100) tel que revendiqué dans n'importe quelle revendication précédente, comprenant en outre :
la force est une force de compression agissant de façon substantiellement perpendiculaire à la première surface et la surface supplémentaire et/ou une force latérale agissant de façon substantiellement parallèle à la première surface et la surface supplémentaire de façon à créer une force de cisaillement entre la première surface et la surface supplémentaire.

14. Le pansement pour plaie (100) de la revendication 12, dans lequel l'orifice d'aspiration (150) comprend en outre :
une surface de scellement (152) destinée à sceller l'orifice d'aspiration (150) sur le pansement pour plaie (100) ;
une portion de raccordement (154) destinée à raccorder l'orifice d'aspiration (150) à une source de pression négative, et
une portion de corps hémisphérique (156) disposée entre la surface de scellement (152) et la portion de raccordement (154) ;
la surface de scellement (152) comprenant un flasque qui fournit une zone plate pour fournir une bonne étanchéité lorsque l'orifice (150) est scellé à la couche de couverture (140).

15. Le pansement pour plaie (100) tel que revendiqué dans n'importe quelle revendication précédente, dans lequel la couche de transmission (105) est plus petite que la couche absorbante (110).

16. Le pansement pour plaie (100) tel que revendiqué dans n'importe quelle revendication précédente, comprenant en outre :
sous une force de compression, la couche absorbante (105) diminue en épaisseur.

17. Le pansement pour plaie (100) tel que revendiqué dans les revendications 1 à 16, comprenant en outre :
sous une force de compression, la couche absorbante (110) diminue en épaisseur et la force de compression agit pour décaler la première surface et la surface supplémentaire de la couche de transmission (105).
